# EUROPEAN PATENT APPLICATION

(11) **EP 3 950 023 A1**
(43) Date of publication of application: **09.02.2022**
(21) Application number: 19923535.9
(22) Date of filing: 02.04.2019
(51) Int. Cl.: A61M 5/142, A61M 5/172

(54) **MONITOR, AND METHOD FOR COMBINED DISPLAY OF PHYSIOLOGICAL SIGN PARAMETERS AND MEDICATION INFORMATION FOR SAME**

(71) Applicant: Shenzhen Mindray Bio-Medical Electronics Co., Ltd., Shenzhen, Guangdong 518057 (CN)
(72) Inventor: TAN, Lin, Shenzhen, Guangdong 518057 (CN); PAN, Ruiling, Shenzhen, Guangdong 518057 (CN); CEN, Jian, Shenzhen, Guangdong 518057 (CN)
(74) Representative: KIPA AB
(86) International application number: PCT/CN2019/080995
(87) International publication number: WO 2020/199119

(57) **Abstract**

Disclosed are a monitor (10), and a method for the combined display of physiological sign parameters and medication information applied to the monitor (10). The monitor (10) comprises a physiological data monitoring device (11), a processor (12), a display screen (13), and a communication unit (14). The physiological data monitoring device (11) is used for monitoring physiological sign parameters of a patient, and the communication unit (14) is used for receiving medication information from an infusion pump (30). The processor (12) is used for controlling the display screen (13) for associated display of the physiological sign parameters and the medication information in the monitor (10). Therefore, the correlation between the physiological sign parameters and the medication information can be clinically analyzed, and this is helpful for analyzing the effects of medication in order to facilitate clinical administration.

## Description

### TECHNICAL FIELD

The disclosure relates to the field of medical monitoring, and in particular to a monitor, and a method for combined display of physiological sign parameters and medication information for same.

### BACKGROUND

During the course of a patient's medication, there are many drugs that affect changes of the patient's physiological sign parameters. Under the action of efficacy of drugs, some changes in vital signs are rapid and remarkable, while other changes are gradual. A physician evaluates the effect of the drugs based on the changes in the patient's physiological parameters, then determines whether the medication strategy needs to be adjusted, and then adopts a more appropriate treatment strategy.

However, a current monitoring system and an infusion system work independently of each other, and information of the monitoring system and the infusion system is not well integrated for use. Consequently, medical staff can obtain related information only from respective outputs of the two systems, and then think about combining the information of the two systems by themselves, so as to analyze a relationship between medication and changes in the patient's physiological parameters. Such an association is extremely implicit and inaccurate. In many clinical drug research projects, researchers have to create a table by themselves to record drug information along with physiological parameter values and then draw a graph to facilitate analysis of a change trend of the patient's physiological parameters under the action of drugs.

### SUMMARY

The disclosure provides a monitor, and a method for combined display of physiological sign parameters and medication information for same, so as to solve the above technical problem.

An embodiment of the disclosure provides a monitor including a physiological data monitoring apparatus, a processor, a display, and a communication unit, where the physiological data monitoring apparatus is configured to monitor physiological sign parameter(s) of a patient, the communication unit is configured to receive medication information from an infusion pump, and the processor is configured to control the display to display the physiological sign parameter(s) and the medication information in association.

An embodiment of the disclosure further provides a method for combined display of physiological sign parameter(s) and medication information, where the method is applied to a monitor and includes the following steps: monitoring physiological sign parameter(s) of a patient; receiving medication information from an infusion pump; and performing associated display of the physiological sign parameter(s) and the medication information on the monitor.

According to the monitor and the method for combined display of physiological sign parameters and medication information for same, the physiological sign parameters of the patient and the medication information from the infusion pump are combined for associated display on the monitor to reflect impact of medication on the physiological sign parameters, thereby facilitating analysis of the effects of drugs and bedside medication administration.

### BRIEF DESCRIPTION OF THE DRAWINGS

In order to more clearly describe the technical solutions in the embodiments of the disclosure or in the prior art, the drawings required for describing the embodiments or the prior art are briefly described below. Apparently, the drawings in the following description show only some of the embodiments of the disclosure, and those of ordinary skill in the art may still derive other drawings from these drawings without creative efforts.
FIG. 1 is a schematic diagram of modules of a monitor and an infusion pump connected over a network according to an embodiment of the disclosure.
FIG. 2 is a schematic diagram showing combined display of physiological sign parameters and medication information of an infusion pump by a monitor according to an embodiment of the disclosure.
FIG. 3 is a diagram of state examples for a pump status according to an embodiment of the disclosure.
FIG. 4 is a schematic diagram of combined display of physiological sign parameters and medication information that are associated based on time according to an embodiment of the disclosure.
FIG. 5 is a schematic diagram of a watch window for assisting an operator in determining whether a medication administration flow rate is appropriate according to an embodiment of the disclosure.
FIG. 6 is a schematic diagram of combined display of physiological sign parameters and medication information that are associated based on distribution according to an embodiment of the disclosure.
FIG. 7 is a max-min-average trend chart according to an embodiment of the disclosure.
FIG. 8 is a principle diagram of removing an abnormal value according to an embodiment of the disclosure.
FIG. 9 is an example diagram of a statistical result of a liquid infusion volume according to an embodiment of the disclosure.
FIG. 10 is a complete example diagram of combined display of physiological sign parameters and medication information of an infusion pump according to an embodiment of the disclosure.
FIG. 11 is a schematic flowchart of a method for combined display of physiological sign parameters and medication information of an infusion pump according to an embodiment of the disclosure.

### DESCRIPTION OF THE PREFERRED EMBODIMENTS

The technical solutions of the embodiments of the disclosure are described below clearly and comprehensively in conjunction with accompanying drawings of the embodiments of the disclosure. Apparently, the embodiments described are only some of, rather than all of, the embodiments of the disclosure. Based on the embodiments in the disclosure, all other embodiments derived by those of ordinary skill in the art without creative efforts shall all fall within the scope of protection of the disclosure.

It can be understood that the terms in the description and the claims of the disclosure as well as the above accompanying drawings are only used to describe specific embodiments, and are not intended to limit the disclosure. The terms "first", "second", etc. in the description and the claims of the disclosure as well as the above accompanying drawings are used to distinguish different objects, rather than to describe a specific order. Singular forms "a/an" and "the" are intended to include a plural form unless stated explicitly otherwise. The terms "including" and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these processes, methods, products, or devices. In addition, the disclosure may be implemented in various forms and is not limited to the embodiments described in the embodiments.

Preferred embodiments of the disclosure are subsequently described in the description, but the description is for the purpose of explaining the general principles of the disclosure and is not intended to limit the scope of the disclosure. The scope of protection of the disclosure is defined by the appended claims.

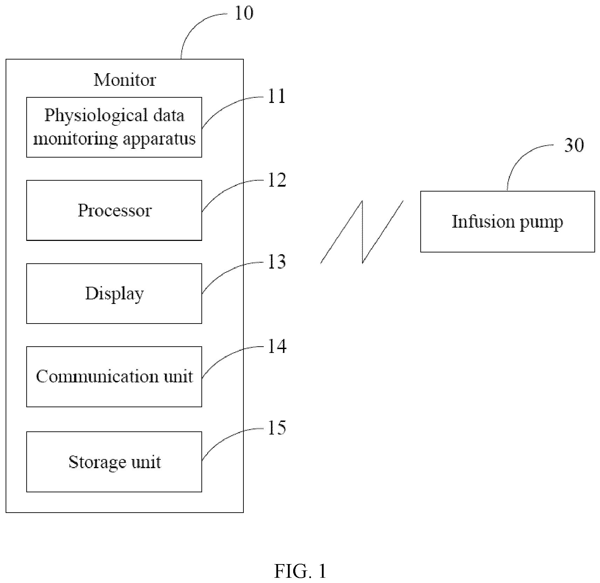

Referring to FIG. 1, FIG. 1 is a schematic diagram of modules of a monitor 10 and an infusion pump 30 connected over a network according to an embodiment of the disclosure. The monitor 10 includes a physiological data monitoring apparatus 11, a processor 12, a display 13, and a communication unit 14. The processor 12 establishes a communication connection to each of the physiological data monitoring apparatus 11, the display 13, and the communication unit 14. Specifically, the physiological data monitoring apparatus 11 is an apparatus for monitoring a heart rate, a respiration rate, blood oxygen, blood pressure, body temperature, etc. of a patient. The processor 12 may be a central processing unit (CPU) or another general-purpose processor, a digital signal processor (DSP), an application-specific integrated circuit (ASIC), a field-programmable gate array (FPGA), or another programmable logic device, a discrete gate or transistor logic device, a discrete hardware component, etc. The general-purpose processor may be a microprocessor, or the general-purpose processor may be any conventional processor, etc. The display 13 is various screens that can be used for information display, for example, an LCD screen. The communication unit 14 may be, but is not limited to, a wired communication device and a wireless communication device, where the wireless communication device may be Bluetooth, near-field communication, wireless fidelity (Wi-Fi), and a telemetry antenna, for example, a wireless medical telemetry service (WMTS) antenna.

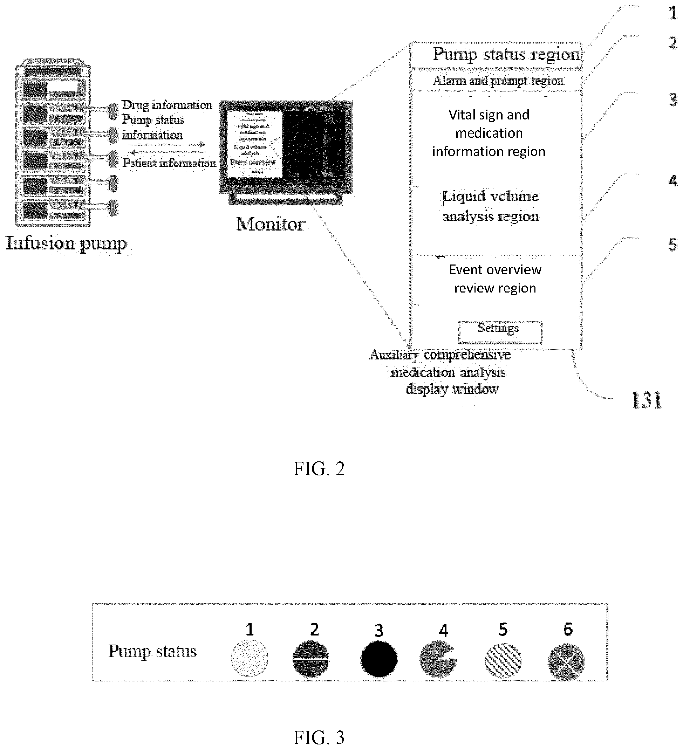

Referring also to FIG. 2, FIG. 2 is a schematic diagram showing combined display of physiological sign parameters and medication information of an infusion pump by a monitor according to an embodiment of the disclosure. The physiological data monitoring apparatus 11 is configured to monitor the physiological sign parameters of a patient. The communication unit 14 is configured to receive the medication information from the infusion pump 30. The processor 12 is configured to control the display 13 to perform associated display of the physiological sign parameters and the medication information.

The medication information includes at least one of a flow rate of a drug, a dose, a concentration, a cumulative infusion liquid volume, and a physiological sign parameter that is mainly affected during the use of the drug, where the physiological sign parameter that is mainly affected during the use of the drug is at least one of the monitored physiological sign parameters.

Specifically, in one embodiment, the monitored physiological sign parameters of the patient include, but are not limited to, a heart rate, a respiration rate, blood oxygen, blood pressure, and body temperature. The infusion pump 30 includes, but is not limited to, a transfusion pump and a syringe pump. The infusion pump 30 establishes a network connection to the monitor 10 in a wired or wireless manner. In this way, the infusion pump 30 sends pump status information and the medication information to the monitor 10. The communication unit 14 of the monitor 10 receives the pump status information and the medication information. The pump status information includes, but is not limited to: a pump start/pause/stop state, a normal or exceptional state, and a pump's access door open/closed state. The medication information includes, but is not limited to: a pump number, a drug name, a drug label color, a flow rate, a dose concentration, a remaining liquid volume, a cumulative infusion liquid volume, a working mode, and a physiological sign parameter that is mainly affected (for example, blood pressure mainly affected by vasopressor drugs). When receiving the pump status information and the medication information from the infusion pump 30, the processor 12 at least controls the display 13 to perform associated display of the physiological sign parameter that is mainly affected during the use of the drug and the medication information.

Therefore, the processor 12 of the monitor 10 can control the display 13 to perform the associated display of the physiological sign parameters of the patient and the medication information, such that an association between the physiological sign parameters and the medication information is presented in a more comprehensible form, to help physicians analyze an association between a medication administration process and fluctuation of a vital sign parameter, and also make it convenient for nurses to perform bedside medication administration.

It can be understood that, in one embodiment, when the monitor 10 and the infusion pump 30 establish a wired or wireless network connection, the infusion pump 30 can also obtain patient information from the monitor 10. The patient information includes, but is not limited to: a patient ID number, a medical record number, a bed number, name, gender, height, weight, age, etc. Therefore, the patient information and the medication information can be associated and stored in the infusion pump 30.

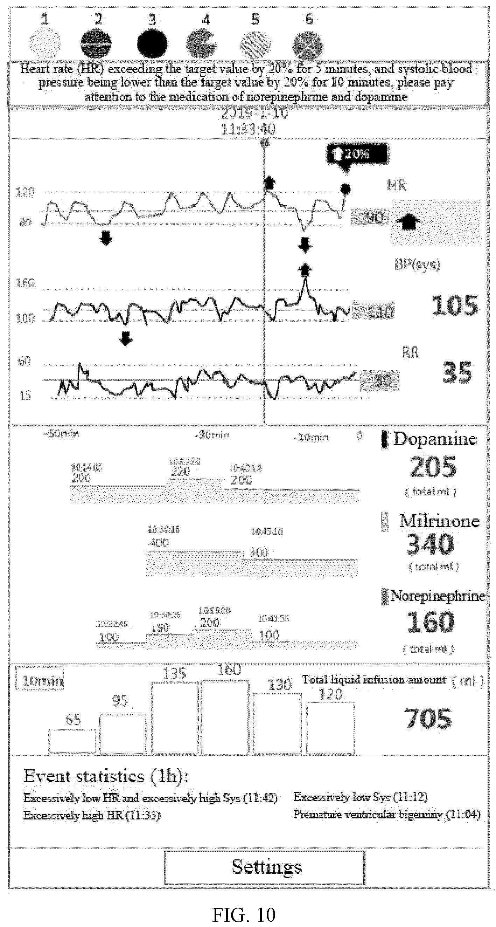

Specifically, the processor 12 controls the display 13 to display an auxiliary comprehensive medication analysis display window 131. The auxiliary comprehensive medication analysis display window 131 includes at least a pump status area 1, an alarm and prompt area 2, a vital sign and medication information display area 3, a liquid volume analysis area 4, and an event overview area 5. FIG. 10 is a complete example diagram of associated display of physiological sign parameters and medication information of an infusion pump according to an embodiment of the disclosure. This is described in detail below.

### Pump status area 1

In one embodiment, the processor 12 controls the display 13 to display received information about a pump status of each pump of the infusion pump 30 in the pump status area 1, and the pump status specifically includes, but is not limited to: started, paused, stopped, accessed, running out of drugs, and faulty. Each pump state is indicated by a corresponding shape, color, and/or character, which specifically includes, but is not limited to, different pump states indicated by graphic transformation, color changes, or text changes. For example, in one embodiment, as shown in FIG. 3, a pump state of a pump 1 is normal in operation; a pump state of a pump 2 is paused in infusion, in which case an operator may be dealing with air bubbles in a liquid path and temporarily stops medication administration; a pump state of a pump 3 is stopped, indicating that this pump no longer performs medication administration; a pump state of a pump 4 is accessed, indicating that an access door of this pump is opened; a pump state of a pump 5 is running out of drugs, which reminds the operator to replenish drugs in time or prepare to end the infusion; and a pump state of a pump 6 is faulty, indicating that a current fault of the pump 6 needs to be dealt with in time, otherwise it affects the normal medication administration. It can be understood that in other embodiment variants, a pump state of each pump can also be indicated by a combination of one or more of graphic transformation, color transformation, text transformation, graphic blinking, light blinking, and sound transformation. Especially in the pump state of running out of drugs or in the faulty state, a status alarm is issued by a combination of one or more of graphic transformation, color transformation, text transformation, graphic blinking, light blinking, and sound transformation, so as to more effectively remind the operator to deal with it.

Further, the monitor 10 further includes a storage unit 15. The storage unit 15 is electrically connected to the processor 12. The storage unit 15 may include a high-speed random access memory, and may further include a non-volatile memory such as a hard disk, an internal memory, and a removable hard disk, a smart media card (SMC), a secure digital (SD) card, a flash card, a plurality of disk storage devices, a flash memory device, or another volatile solid-state storage device. When the display 13 displays the pump status information and the status alarm, the processor 12 further associates the pump status information and the status alarm with the patient information and controls the storage unit 15 to store association data of the pump status information, the status alarm, and the patient information in patient data to serve as event reference information for querying the patient data.

### Alarm and prompt area 2

The processor 12 is further configured to, when unusual fluctuation occurs in any physiological sign parameter monitored by the physiological data monitoring apparatus 11, control the display 13 to mainly present, in the alarm and prompt area 2, prompt information that is related to a medication effect. In one embodiment, it is controlled to generate the prompt information when unusual fluctuation occurs in any physiological sign parameter monitored by the physiological data monitoring apparatus 11. The physiological sign parameter may be a physiological sign parameter closely related to medication information, or may be a physiological sign parameter that is less related or not related to the medication information such that when unusual fluctuation occurs, the operator is reminded to pay attention to control over a drug usage amount or an injection flow rate closely related to the vital sign parameter.

It can be understood that, in one embodiment, the prompt information includes not only prompt information, but also alarm information. The alarm may be, but is not limited to, a sound alarm signal and a light alarm signal, or may be a voice prompt signal or display signal with corresponding content, etc.

Specifically, in one embodiment, when the use of one or more drugs is related to one or more physiological sign parameters of the patient, it is desired in clinical treatment to stabilize measured values of the physiological sign parameters within a reasonable target range. It can be understood that the operator can define the target range as a target range suitable for the patient based on a physiological condition of the patient. When the measured values of the physiological sign parameters of the patient fluctuate within the target range, it indicates that the fluctuation of the physiological sign parameters is normal; when the measured values of the physiological sign parameters of the patient exceed the target range, it indicates that unusual fluctuation occurs in the physiological sign parameters, and the processor 12 controls to generate prompt information.

In one embodiment, the content of the prompt information includes a combination of one or more of a specific pattern, color, text, voice, and video, that is, the prompt information can be characterized by a specific pattern, color, text, voice, or video. Therefore, the operator can quickly understand the content of the prompt information, which makes it convenient for the operator to perform a next step in time.

It can be understood that, in other embodiments, the processor 12 also controls to issue prompt information when the measured values of other physiological sign parameters of the patient that are not associated with or have little association with the use of drug exceed a target range corresponding to the measured values of the physiological sign parameters.

Further, in one embodiment, the storage unit 15 stores a predefined correspondence between a fluctuation range of each physiological sign parameter and a prompt level. The processor 12 determines and controls to generate prompt information corresponding to the prompt level according to the correspondence and a current range of unusual fluctuation of the physiological sign parameter. For example, the level may be, but is not limited to, high, medium, low, etc. Therefore, the operator can tell a current prompt level, for example, high, medium, and low, according to current prompt information in a working process, and give priority to a patients with a high prompt level.

For example, in one embodiment, a vasoactive drug, norepinephrine, is administered to a patient with hypotension, and norepinephrine increases blood pressure of the patient. Clinically, for the patient with hypotension, most operators give a blood pressure target range according to the condition of the patient, for example, maintaining blood pressure within 20 mmHg fluctuation at 120 mmHg/85 mmHg (systolic/diastolic blood pressure). Once norepinephrine is administered to the patient, the patient's blood pressure reaches the target range within a period of time, which is followed by a period of the continuous use of drug. It can be understood that the flow rate of the infusion pump 30 during this period of time does not change freel,m y in most cases. After a period of time, if the patient's blood pressure falls again and falls out of the target range, that is, systolic blood pressure is lower than 100 mmHg, or diastolic blood pressure is lower than 65 mmHg, the processor 12 controls to generate event information, for example, "the systolic blood pressure or the diastolic blood pressure is lower than the target range, pay attention to the amount of used norepinephrine", and controls to display same in the alarm and prompt area 2. Otherwise if the patient's blood pressure is higher than the target range, that is, the systolic blood pressure is higher than 140 mmHg, or the diastolic blood pressure is higher than 105 mmHg, the processor 12 also controls to generate event information, for example, "the systolic blood pressure or the diastolic blood pressure is higher than the target range, pay attention to the amount of used norepinephrine", and controls to display same in the alarm and prompt area 2. The processor 12 further provides prompt information that matches a prompt level of the above event information according to the prompt level, and at the same time, controls to highlight the norepinephrine in the display 13, to prompt the operator to consider a use strategy of the norepinephrine.

For another example, the operator uses an anti-arrhythmic drug for a patient with arrhythmia problems. The drug lidocaine is usually injected for the patient with arrhythmia such as ventricular premature beats or ventricular tachycardia. Under normal conditions, the patient's related arrhythmia problems disappear after the use of the lidocaine for a period of time. If such arrhythmia problems still exist, or such arrhythmia problems occur less frequently but still exist, it means that the drug is not effective for the patient, and other anti-arrhythmic drugs should be considered. The processor 12 controls to generate event information, for example, "ventricular tachycardia, pay attention to the use of lidocaine", and controls the display 13 to display the event information in the alarm and prompt area 2 to inform the operator in time.

It can be understood that the above target range may also be replaced with a proportion by which a target value is exceeded, or an absolute value by which a target value is exceeded, for example, twenty percent of the target value.

Further, in one embodiment, the storage unit 15 further stores a predefined duration threshold for triggering generation of a prompt information by unusual fluctuation of each physiological sign parameter. The processor 12 controls to generate prompt information when duration of unusual fluctuation of any physiological sign parameter reaches a corresponding duration threshold. For example, a five-minute duration constraint is set for the event that the systolic blood pressure is higher than 140 mmHg or the diastolic blood pressure is higher than 105 mmHg, such that the processor 12 controls to generate event information only when the systolic blood pressure is higher than the threshold 140 mmHg for five minutes or the diastolic blood pressure is higher than 105 mmHg for five minutes. In this way, the interference caused by occasional transient fluctuation can be effectively avoided.

Further, in one embodiment, when it is determined, according to unusual fluctuation of a physiological sign parameter, that the current flow rate of the drug needs to be controlled, the processor 12 is further configured to generate a drug flow rate control instruction, and send the drug flow rate control instruction to the infusion pump 30 using the communication unit 14, so that the infusion pump 30 controls the flow rate of the drug of the infusion pump in response to the drug flow rate control instruction. Therefore, when it is determined, according to the unusual fluctuation of the physiological sign parameter, that the current flow rate of the drug needs to be controlled, the flow rate of the drug can be controlled in time, the workload of the operator can be reduced, and the intelligent management of patient monitoring and infusion can be achieved.

Further, in one embodiment, when it is determined, according to unusual fluctuation of a physiological sign parameter, that infusion needs to be started or stopped currently, the processor 12 is further configured to generate an infusion start or stop instruction, and control the communication unit 14 to transmit the infusion start or stop instruction to the infusion pump 30, so that the infusion pump 30 starts or stops the infusion in response to the instruction. Therefore, when it is determined, according to the unusual fluctuation of the physiological sign parameter, that the infusion needs to be started or stopped currently, a corresponding control operation of starting or stopping the infusion can be immediately performed in response to the instruction, thus avoiding accidents, and achieving the intelligent management of patient monitoring and infusion.

### Vital sign and medication information display area 3

The processor 12 controls the display 13 to perform combined display of the physiological sign parameters and the medication information in the vital sign and medication information display area 3, so that the medical staff can view an association between a medication administration process and fluctuation of vital sign parameters, and help the medical staff pay attention to the effect of the use of drugs on the stability of the patient's vital sign parameters.

In one embodiment, the physiological sign parameters and the medication information are displayed in association in at least two manners. In a first manner: the two are displayed by time-based association, which means that a correlation between a physiological sign parameter trend and a medication flow rate trend or a dose concentration trend is analyzed over time. For example, the processor 12 can be configured to control the display 13 to perform associated display of the physiological sign parameters and the medication information by: associating, by the processor 12, the physiological sign parameters with the medication information in time, and then controlling the display 13 to display the correlation between the physiological sign parameter trend and the medication flow rate trend or a dose concentration trend over time. In a second manner, the physiological sign parameter and the medication information are displayed by distribution-based association, which means comparison of distribution of measured values of the physiological sign parameters in a period of time before and after medication. Specifically, the processor 12 can be configured to control the display 13 to perform associated display of the physiological sign parameters and the medication information by: associating, by the processor 12, the physiological sign parameters within a preset time period before medication with the physiological sign parameters within a preset time period after the medication, and then controlling the display 13 to display an association between the physiological sign parameters and the use of such the drug.

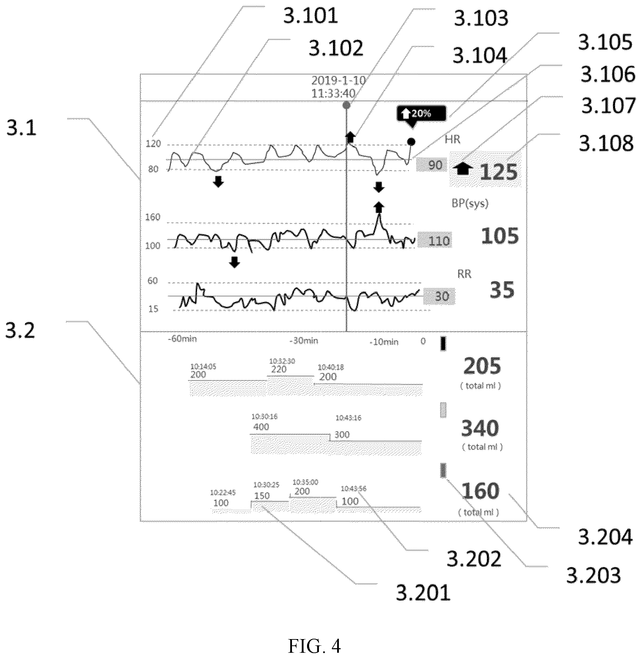

As shown in FIG. 4, FIG. 4 is a schematic diagram of combined display of physiological sign parameters and medication information that are associated based on time. An area 3.1 is an area for a trend chart of measured values of the physiological sign parameters. An area 3.2 is an area for a trend chart area of a drug flow rate (or dose concentration). In this figure, an area for a one-hour trend chart is displayed. It can be understood that a time interval length of the trend chart can be customized according to the needs of an operator.

In this example, in the area 3.1, a ruler line 3.101 can be maximum and minimum alarm limit thresholds, or a target range threshold; a trend curve 3.102 of a physiological sign parameter reflects a change of the physiological sign parameter over time; the operator can move a cursor 3.103 on a timeline to point to a certain time point; an element 3.104 refers to an event that a measured value exceeds the threshold in the past; an element 3.105 refers to an event that a measured value of the physiological sign parameter exceeds the threshold currently; an element 3.106 is a target value ruler which can be provided when the operator defines a specific target value instead of the target range2021/11/2, and then the processor 12 can calculate the target range threshold according to the defined threshold (such as a change of 20%); an arrow of an abnormality indicator icon 3.107 indicates the nature of an abnormality, that is, whether it is higher than the threshold or lower than the threshold, where the manner of indicating the abnormality is not limited to an icon, and the abnormality can also be indicated by color, or blinking color and measured value; an element 3.108 is a measured value at the time point selected by the cursor. In the area 3.1 of FIG. 4, one-hour trend curves of three physiological sign parameters, namely, a heart rate (HR), systolic blood pressure (BP-sys), and a respiratory rate (RR) are given. The operator can select several physiological sign parameters that need to be paid attention to according to his/her own needs.

In the area 3.2, an element 3.201 is a flow rate trend of the drug; an element 3.202 is a time at which the flow rate changes; and an element 3.203 is a drug name and a drug label color. Color-coded labels are usually used clinically to distinguish different types of drugs, for example, violet for vasopressor drugs and warm red for muscle relaxant drugs. An element 3.204 is a cumulative liquid volume of the drug in a time period. The flow rate trend may alternatively be replaced by a dose concentration trend. In the area 3.2 of FIG. 4, flow rate trend curves of the three drugs dopamine, milrinone, and norepinephrine obtained by the monitor from the integrated infusion pump 30 are given. According to the number of integrated infusion pumps 30 and a specific drug name, the monitor can display flow rate (or dose concentration) trends of more drugs.

The measured values of the physiological sign parameters and the drug flow rate trend are displayed on a timeline. Since a concentration of a prepared drug solution is fixed, a change of the flow rate means a change of a drug amount in a unit time, and a relationship between a segment of unusual fluctuation of the physiological sign parameter and the drug flow rate can be easily seen. When the cursor moves to a certain event position, a measured value of the physiological sign parameter and the drug flow rate information at that time point are displayed.

When the operator adjusts the flow rate of the medication administration, the effects of some drugs on the physiological sign parameters are very sensitive with a rapid response. For example, the effects of vasoactive drugs (for example, dopamine, milrinone, norepinephrine, etc.) on the blood pressure and the heart rate are remarkable and sensitive, and sedation and analgesia drugs (for example, lidocaine, as well as quinidine, metoprolol, amiodarone, verapamil, etc.) have remarkable and sensitive effects on consciousness, followed by the heart rate and the blood pressure. After adjusting the flow rate of the medication administration, the operator waits for a period of time beside the infusion pump 30 to observe a change trend of the patient's related physiological sign parameters and determine whether a current flow rate of the medication administration is appropriate. If the current flow rate of the medication administration is inappropriate, the current flow rate may be further adjusted, and if the current flow rate of the medication administration is appropriate, the current flow rate may be maintained.

Further, when at least one of the flow rate of the drug and the dose concentration changes or a new drug is added, the processor 12 controls to generate a corresponding parameter curve or a mathematical expression of parameter value fluctuation, and controls the display 13 to display the parameter curve or the mathematical expression of parameter value fluctuation. The parameter curve is a curve formed by fluctuation of the physiological sign parameter over time. The mathematical expression of the parameter value fluctuation is a mathematical expression of the curve formed by the fluctuation of the physiological sign parameter, for example, a mathematical expression that forms the curve, or a key feature value that forms the curve, such as a slope.

Further, in one embodiment, when it is determined that the flow rate of the drug increases, the processor 12 controls the display 13 to display a watch window, where in the watch window, a change trend chart of physiological sign parameters closely related to the drug versus the flow rate of the drug within a preset time period is displayed, with a current flow rate value of the drug highlighted.

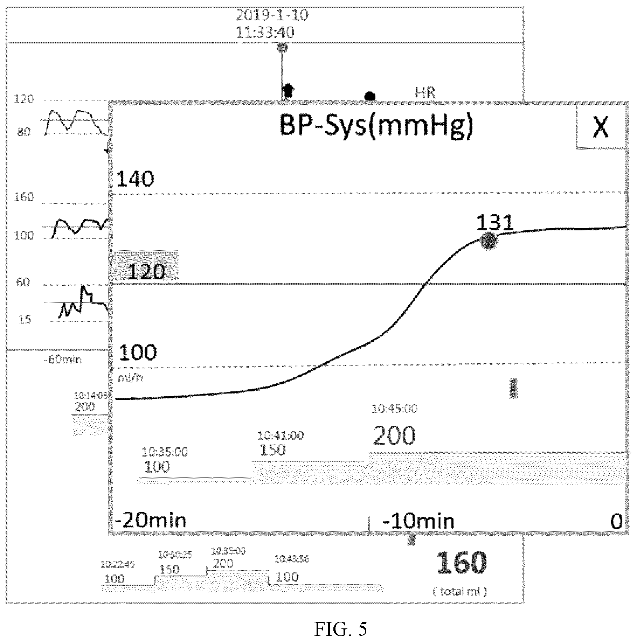

As shown in FIG. 5, in one implementation example, a change in a flow rate of norepinephrine is used to show a design that assists the operator in determining whether a flow rate of medication administration is appropriate. When determining that the flow rate of norepinephrine administration increases, the processor 12 controls the display 13 to display a watch window to display a trend chart of changes in the flow rate of the norepinephrine and the blood pressure within a preset event, with a current flow rate value highlighted. At this time, a systolic blood pressure (BP-Sys) trend is acquired with high-precision data, and an acquisition dot frequency is not less than one dot per second, so that changes in the blood pressure trend can be refreshed in time. When the flow rate is 100 mL/h, the patient's blood pressure is already below the target range, and then the operator increases the flow rate to 150 mL/h; the patient's blood pressure rises to a certain extent, but the rise is slow and not to the expectations, and then the operator further increases the flow rate to 200 mL/h; the operator empirically determines that a rise slope of a blood pressure curve is ideal at this time, and the blood pressure rise slows down shortly and begins to maintain in a relatively stable state. The systolic blood pressure value at a stable inflection point is 131 mmHg, reaching the blood pressure target, and thus the medication administration maintains at the flow rate of 200 mL/h subsequently.

Further, in one embodiment, the processor 12 is further configured to close the watch window 11 in response to a close operation of a user, or the processor 12 controls to close the watch window 11 when the flow rate of such a drug no longer changes within the preset time period.

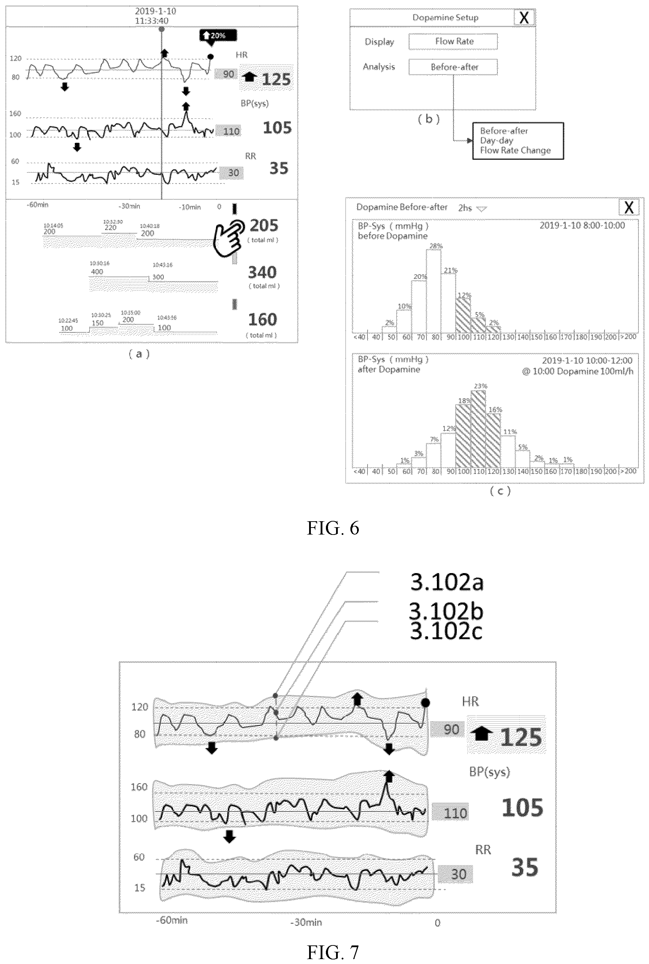

Further, in one embodiment, as shown in FIG. 6, FIG. 6 is a schematic diagram of combined display of physiological sign parameters and medication information that are associated based on distribution. In response to an operation of clicking a dopamine area in a window (a), the processor 12 controls to open a "Dopamine Setup" menu (b), and controls to select whether to display the flow rate trend or the dose concentration trend in the menu, and select an analysis method. The analysis method includes, but is not limited to, before-after medication comparison, day-day comparison, and flow rate change. A window (c) is an analysis result of the before-after medication of dopamine. In the window (c), dopamine was administered to the patient at 2019-1-10 10:00. An upper part of the window (c) is a statistical distribution diagram of the measured values of the systolic blood pressure 2 hours before the use of the drug dopamine, and a lower part is a statistical distribution diagram of the measured values of the systolic blood pressure 2 hours after the use of the drug dopamine. The statistical duration can be modified by the operator. It can be seen from the figure that with the use of the drug dopamine, the proportion of the original hypotensive patient's blood pressure in the target range of the blood pressure began to increase. It can also be understood that the blood pressure of the patient is mainly distributed in 61 mmHg to 90 mmHg (accounting for 69%, in other words, 69% of the blood pressure falling within such a blood pressure range in terms of time) 2 hours before the medication, while only 19% of the blood pressure falls within the target range. With the use of the drug, the proportion of the patient's blood pressure falling within the target range within 2 hours after the medication increased to 57%.

Further, in one embodiment, the processor 12 can be configured to control the display 13 to perform associated display of the physiological sign parameters and the medication information by: presenting the physiological sign parameters by a max-min-average trend chart, where the max-min-average trend chart is formed by: obtaining maximum values, minimum values, and average values in a plurality of values of the physiological sign parameters measured in each unit time within the preset time period, and then connecting all the maximum values, all the minimum values, and all the average values within the preset time period to form respective curves, so as to form the max-min-average trend chart representing the physiological sign parameter trend, where the preset time period includes multiple unit times.

In addition to the average value trend chart (element 3.102) shown in FIG. 4, which is a line obtained by connecting the average values in the unit times, a trend chart of the measured values of the physiological sign parameters in the area 3.1 may be a maximum-minimum value trend chart. With the development of high-speed data collection, the physiological data monitoring apparatus 11 can obtain a plurality of data values within several extremely small time periods. However, limited by the actual number of pixels of the display 13 in a limited area, it may not be viable to show all points truthfully. In this case, one pixel may represent one unit of time (such as 1 second, 1 minute, and 5 minutes) to obtain three of the values measured in the units of time, namely, the maximum value, the minimum value, and the average value. Then, all the maximum values, all the minimum values, and all the average values within the preset time period are connected to for respective curves, to form the max-min-average trend chart.

FIG. 7 is a max-min-average trend chart according to an embodiment of the disclosure. Elements 3.102a, 3.102b, and 3.102c are three values in the same time unit. The element 3.102a is a maximum value in the unit time, the element 3.102b is an average value in the unit time, and 3.102c is a minimum value in the unit time. In this way, the operator can clearly see a fluctuation range of the patient's vital sign values, which has good guiding significance for the use of an area to display a trend curve for a long time, and avoids a case where a fluctuation status of the patients with frequently fluctuated vital sign values is ignored in the case of only an average value.

However, no matter it is the average value trend curve or the maximum-minimum value trend curve mentioned above, an interference removal method can also be used to remove extremely abnormal measured values before averaging or selection of the maximum and minimum values is performed. Clinically extremely abnormal measured values do not reflect a real condition of the patient's vital signs, and instead, interference introduced by some factors leads to abnormally large deviation from the measured values.

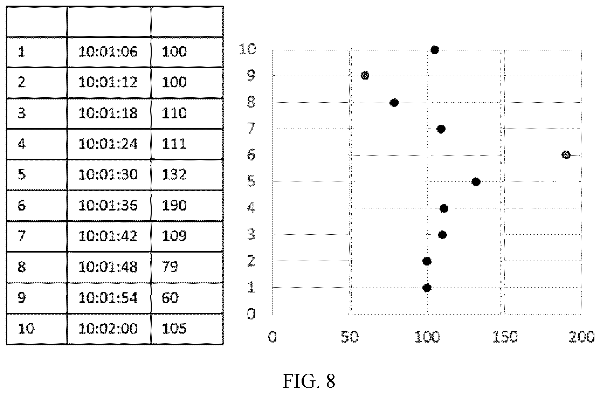

FIG. 8 is a principle diagram of removing an abnormal value according to an embodiment of the disclosure. Taking 10 pieces of heart rate data collected by the monitor within 1 minute as an example, the data numbered 6 in the 10 pieces of data is 190, which is abnormally high. Therefore, the 6^{th} piece of data is removed, only 9 pieces of data between the two dashed lines are used to calculate an average value, the data numbered 5 is selected as a maximum value (132), and the data numbered 8 is selected as a minimum value (79). The dashed lines can be selected according to fluctuation rules of the physiological sign parameters of a human body, and it may be considered that a proportion of abnormal values shall not exceed the percentage of a data amount in a unit time, such as 90%.

Further, in one embodiment, the monitor collects statistics on infusion amounts of different drugs infused into the body of the monitored object in each time period to obtain the cumulative infusion amounts of different drugs in different time periods. Therefore, an overall trend of an infused volume for the patient, such as a liquid infusion trend, within 1 hour, 8 hours, and 24 hours, can be observed.

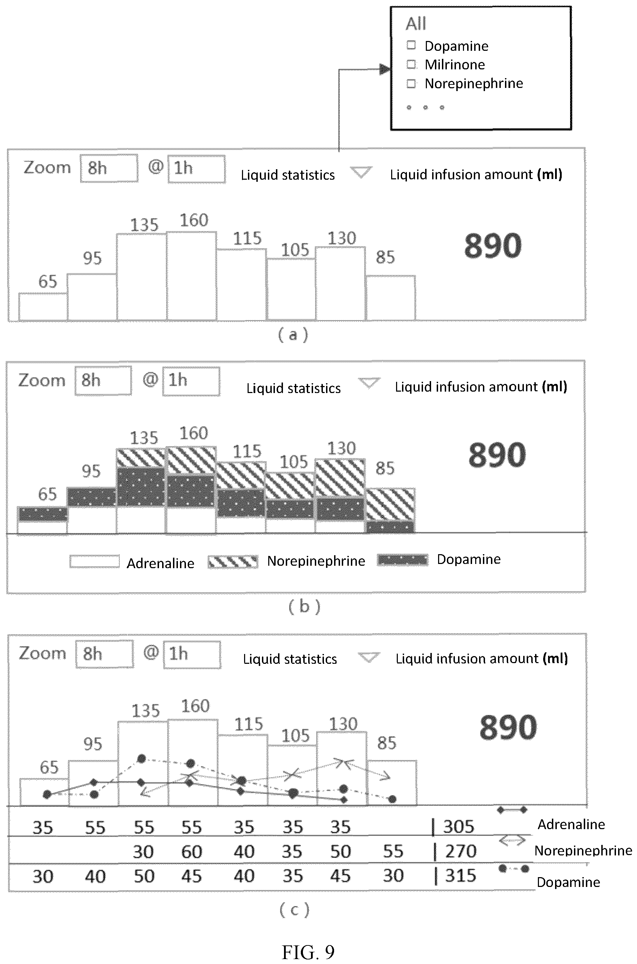

FIG. 9 is an example diagram of a liquid infusion statistical result, showing the liquid infusion within 8 hours. The operator can select a time period and a sub-time period represented by each histogram. A time period shown in FIG. 9(a) is 8 hours, and each histogram represents 1 hour. The operator can also manually select drugs that need to be performed with the statistical collection, for example, all or several drugs, such as statistical collection on an adrenaline dose/liquid infusion amount, on a dopamine dose/liquid infusion amount, or on adrenaline, norepinephrine, and dopamine dose /liquid infusion amounts shown in FIGS. 9(b) and 9(c). In FIG. 9(b), trend display for different drugs is added, and in FIG. 9(c), a cumulative trend of different drugs and a cumulative infusion amount in a unit time are added.

### Event overview area 5

Further, the processor 12 collect statistics on types of abnormality events, the number of occurrences of abnormality events, and key indicators in abnormality events within a preset time period, and controls the display 13 to display, in the event overview area 5, the types of abnormality events, the number of occurrences of abnormality events, and the key indicators in the abnormality events within the preset time period. For example, statistics on the total number of occurrences of arrhythmia events within 8 hours, and the number of occurrences of different arrhythmia events may be collected, such as "ventricular fibrillation (1)" and "excessively high HR (3)". At the same time, detailed information, such as "ventricular fibrillation @ 10:54 for 55 seconds", "excessively high HR @ 10:03, @ 10:18, and @ 10:41, Max 135, and Min 87" can be viewed. With the information, the operator can view details of the event in the vital sign and medication information display area 3, as well as a trend of the parameters for a period of time before and after the event.

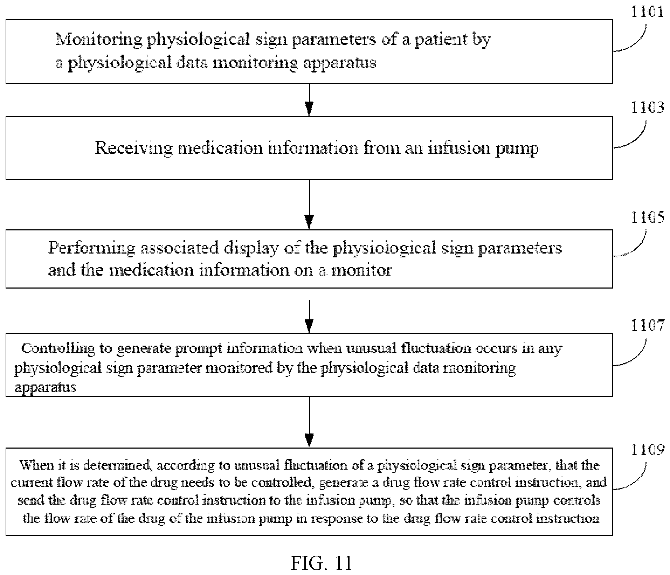

Referring to FIG. 11, FIG. 11 is a flowchart of a method, applied to a monitor, for combined display of physiological sign parameters and medication information according to an embodiment of the disclosure. The execution order of the method is not limited to the order shown in FIG. 11. The method for the combined display of the physiological sign parameters and the medication information includes the steps as follows.

At step 1101: physiological sign parameters of a patient are monitored. Specifically, the physiological data monitoring apparatus 11 of the monitor 10 monitors the physiological sign parameters of the patient.

At step 1103: medication information from the infusion pump 30 is received. Specifically, the communication unit 14 of the monitor 10 receives the medication information from the infusion pump 30.

At step 1105: associated display of the physiological sign parameters and the medication information on the monitor 10 is performed. The processor 12 of the monitor 10 controls to perform associated display of the physiological sign parameters and the medication information on the display 13.

The medication information includes at least one of a flow rate of a drug, a dose concentration, a cumulative infused liquid volume, and a physiological sign parameter that is mainly affected during the use of the drug, and the physiological sign parameter that is mainly affected during the use of the drug is at least one of the monitored physiological sign parameters.

Further, in one embodiment, the method further includes the following step.

At step 1107: it is controlled to generate prompt information when unusual fluctuation occurs in any physiological sign parameter monitored by the physiological data monitoring apparatus 11.

Further, in one embodiment, the step 1107 includes: determining and controlling to generate prompt information corresponding to a prompt level according to a predefined correspondence between a fluctuation range of each physiological sign parameter and a prompt level and a current range of unusual fluctuation of the physiological sign parameter.

Further, in one embodiment, the step 1107 includes controlling to generate the prompt information when duration of unusual fluctuation of any physiological sign parameter reaches a corresponding duration threshold, where the duration threshold is a predefined duration threshold for triggering generation of a prompt information by unusual fluctuation of each physiological sign parameter.

Further, in one embodiment, the method further includes the following step:
At step 1109: when it is determined, according to unusual fluctuation of a physiological sign parameter, that the current flow rate of the drug needs to be controlled, a drug flow rate control instruction is generated, and the drug flow rate control instruction is sent to the infusion pump 30, so that the infusion pump 30 controls the flow rate of the drug of the infusion pump in response to the drug flow rate control instruction.

Further, in another embodiment, the step 1109 may include generating an infusion start or stop instruction when it is determined, according to unusual fluctuation of a physiological sign parameter, that infusion needs to be started or stopped currently, and controlling to send the infusion start or stop instruction to the infusion pump 30, so that the infusion pump 30 starts or stops the infusion in response to the instruction.

Further, in one embodiment, the method further includes a step of:
when at least one of the flow rate of the drug and the dose concentration changes or a new drug is added, controlling to generate and display a corresponding parameter curve or a mathematical expression of parameter value fluctuation.

Further, in one embodiment, the method further includes a step of:
when it is determined that the flow rate of the drug increases, controlling to display a watch window, where in the watch window, a change trend chart of a physiological sign parameter closely related to the drug versus the flow rate of the drug within a preset time period is displayed, with a current flow rate value of the drug highlighted.

Further, in one embodiment, the method further includes a step of:
closing the watch window in response to a close operation of a user; or
controlling to close the watch window when the flow rate of the drug no longer changes within the preset time period.

Further, in one embodiment, the associated display of the physiological sign parameters and the medication information on the monitor includes:
associating the physiological sign parameters with the medication information in time, and then displaying on the monitor a correlation between the physiological sign parameter trend and a medication flow rate trend or a dose concentration trend over time.

Further, in one embodiment, the associated display of the physiological sign parameters and the medication information on the monitor includes:
associating the physiological sign parameters within a preset time period before medication with the physiological sign parameters within a preset time period after the medication, and then displaying on the monitor an association between the physiological sign parameters and the use of the drug.

Further, in one embodiment, the method further includes a step of:
presenting the physiological sign parameters by a max-min-average trend chart.

Further, in one embodiment, the max-min-average trend chart is formed by:
obtaining maximum values, minimum values, and average values in a plurality of values of the physiological sign parameters measured in each unit time within the preset time period; and
connecting all the maximum values, all the minimum values, and all the average values within the preset time period to form respective curves, so as to form the max-min-average trend chart representing a physiological sign parameter trend, where the preset time period includes a number of unit times.

Further, in one embodiment, the method further includes a step of:
collecting time period-based statistics on infusion amounts of different drugs pumped into the body of the patient to obtain cumulative infusion amounts of different drugs in different time periods.

Further, in one embodiment, the method further includes a step of:
collecting statistics on types of abnormality events, the number of occurrences of the abnormality events, and key indicators in the abnormality events within a preset time period, and controlling to display the statistics.

Therefore, the monitor can perform associated display of the physiological sign parameters of the patient and the medication information, such that an association between the physiological sign parameters of the patient and the medication information can be presented in a more comprehensible form, to help physicians analyze an association between a medication administration process and fluctuation of a vital sign value, and also make it convenient for nurses to perform bedside medication administration.

The above embodiments do not constitute a limitation on the scope of protection of the technical solutions. Any modification, equivalent replacement, improvement, etc. made within the spirit and principle of the above embodiments shall fall within the scope of protection of the technical solutions.

## Claims

1. A monitor, **characterized in that** comprising a physiological data monitoring apparatus, a processor, a display, and a communication unit, wherein the physiological data monitoring apparatus is configured to monitor physiological sign parameter(s) of a patient, the communication unit is configured to receive medication information from an infusion pump, and the processor is configured to control the display to perform associated display of the physiological sign parameter(s) and the medication information.

2. The monitor of claim 1, **characterized in that** the medication information comprises at least one of a flow rate of a drug, a dose, a concentration, a cumulative infusion liquid volume, and a physiological sign parameter that can be affected during the use of the drug, and the physiological sign parameter that can be affected during the use of the drug is at least one of the monitored physiological sign parameter(s).

3. The monitor of claim 1, **characterized in that** the processor is further configured to control to generate prompt information when unusual fluctuation occurs in any physiological sign parameter monitored by the physiological data monitoring apparatus.

4. The monitor of claim 3, **characterized in that** further comprising a storage unit, wherein the storage unit stores a predefined correspondence between a fluctuation range of each physiological sign parameter and a prompt level, and the processor determines and controls to generate prompt information corresponding to the prompt level according to the correspondence and a current range of unusual fluctuation of the physiological sign parameter.

5. The monitor of claim 4, **characterized in that** the storage unit further stores a predefined duration threshold for triggering generation of a prompt information by unusual fluctuation of each physiological sign parameter, and the processor controls to generate prompt information when a duration of unusual fluctuation of any physiological sign parameter reaches a corresponding duration threshold.

6. The monitor of claim 3, **characterized in that** content of the prompt information comprises a combination of one or more of a pattern, color, text, voice, and video.

7. The monitor of claim 3, **characterized in that** the processor is further configured to, when it is determined, according to unusual fluctuation of a physiological sign parameter, that a current flow rate of the drug needs to be controlled, generate a drug flow rate control instruction, and control the communication unit to send the drug flow rate control instruction to the infusion pump, so that the infusion pump controls the flow rate of the drug of the infusion pump in response to the drug flow rate control instruction.

8. The monitor of claim 7, **characterized in that** the processor is further configured to, when it is determined, according to unusual fluctuation of a physiological sign parameter, that infusion needs to be started or stopped currently, generate an infusion start or stop instruction, and control the communication unit to send the infusion start or stop instruction to the infusion pump, so that the infusion pump starts or stops the infusion in response to the infusion start or stop instruction.

9. The monitor of claim 2, **characterized in that** when at least one of the flow rate of the drug, the dose and the concentration changes or a new drug is added, the processor controls to generate a corresponding parameter curve or a mathematical expression of parameter value fluctuation, and controls the display to display the parameter curve or the mathematical expression of parameter value fluctuation.

10. The monitor of claim 9, **characterized in that** when it is determined that the flow rate of the drug increases, the processor controls the display to display a watch window, in which a change trend chart of a physiological sign parameter closely related to the drug changing following the flow rate of the drug within a preset time period is displayed with a current flow rate value of the drug highlighted.

11. The monitor of claim 10, **characterized in that** the processor is further configured to close the watch window in response to a close operation of a user, or the processor controls to close the watch window when the flow rate of the drug no longer changes within a preset time period.

12. The monitor of claim 1, **characterized in that** the processor being configured to control the display to perform associated display of the physiological sign parameter(s) and the medication information comprises: associating the physiological sign parameter(s) with the medication information in time, and then controlling the display to display a correlation between the physiological sign parameter(s) and a medication flow rate trend, a dose trend or a concentration trend over time.

13. The monitor of claim 1, **characterized in that** the processor being configured to control the display to perform associated display of the physiological sign parameters and the medication information comprises: associating physiological sign parameter(s) within a preset time period before medication with physiological sign parameter(s) within a preset time period after the medication, and then controlling the display to display an association between the physiological sign parameters and the use of the drug.

14. The monitor of claim 12, **characterized in that** the processor being configured to control the display to perform associated display of the physiological sign parameters and the medication information comprises: controlling to present the physiological sign parameter(s) by a max-min-average trend chart,
wherein the max-min-average trend chart is formed by: obtaining maximum values, minimum values, and average values in a plurality of values of the physiological sign parameter(s) measured in each unit time within the preset time period, and then connecting all maximum values, all minimum values, and all average values within the preset time period to form respective curves, so as to form the max-min-average trend chart representing a physiological sign parameter trend, wherein the preset time period comprises a number of unit times.

15. The monitor of claim 1, **characterized in that** the processor collects statistics on infusion amounts of different drugs infused into a body of the patient in each time period to obtain cumulative infusion amounts of different drugs in different time periods.

16. The monitor of claim 1, **characterized in that** the processor is further configured to collect statistics on types of abnormality events, the number of occurrences of the abnormality events, and key indicators in the abnormality events within a preset time period, and control the display to display the statistics.

17. A method for combined display of physiological sign parameters and medication information, wherein the method is applied to a monitor and comprises the following steps:
monitoring physiological sign parameters of a patient;
receiving medication information from an infusion pump; and
performing associated display of the physiological sign parameters and the medication information on the monitor.

18. The method of claim 17, wherein the medication information comprises at least one of a flow rate of a drug, a dose or concentration, a cumulative infused liquid volume, and a physiological sign parameter that is mainly affected during the use of the drug, and the physiological sign parameter that is mainly affected during the use of the drug is at least one of the monitored physiological sign parameters.

19. The method of claim 17, wherein the method further comprises the following step:
controlling to generate prompt information when unusual fluctuation occurs in any monitored physiological sign parameter.

20. The method of claim 19, wherein controlling to generate prompt information when unusual fluctuation occurs in any monitored physiological sign parameter comprises:
determining and controlling to generate prompt information of a corresponding level according to a predefined correspondence between a fluctuation range of each physiological sign parameter and a prompt level and a range of unusual fluctuation of the current physiological sign parameter.

21. The method of claim 19, wherein controlling to generate prompt information when unusual fluctuation occurs in any monitored physiological sign parameter comprises:
controlling to generate prompt information when duration of unusual fluctuation of any physiological sign parameter reaches a corresponding duration threshold, wherein the duration threshold is a predefined duration threshold for unusual fluctuation of each physiological sign parameter that triggers generation of a prompt.

22. The method of any one of claims 19 to 21, wherein the method further comprises:
when it is determined, according to unusual fluctuation of a physiological sign parameter, that the current flow rate of the drug needs to be controlled, generating a drug flow rate control instruction; and
controlling to send the drug flow rate control instruction to the infusion pump, so that the infusion pump controls the flow rate of the drug of the infusion pump in response to the drug flow rate control instruction.

23. The method of claim 22, wherein the method further comprises:
when it is determined, according to unusual fluctuation of a physiological sign parameter, that infusion needs to be started or stopped currently, generating an infusion start or stop instruction; and
controlling to send the infusion start or stop instruction to the infusion pump, so that the infusion pump starts or stops the infusion in response to the instruction.

24. The method of claim 17, wherein the method further comprises:
when at least one of the flow rate of the drug and the dose or concentration changes or a new drug is added, controlling to generate and display a corresponding parameter curve or a mathematical expression of parameter value fluctuation.

25. The method of claim 24, wherein the method further comprises:
when it is determined that the flow rate of the drug increases, controlling to display a watch window, wherein in the watch window, a change trend chart of a physiological sign parameter closely related to the drug versus the flow rate of the drug within a preset time period is displayed, with a current flow rate value of the drug highlighted.

26. The method of claim 25, wherein the method further comprises:
closing the watch window in response to a close operation of a user; or
controlling to close the watch window when the flow rate of the drug no longer changes within the preset time period.

27. The method of claim 17, wherein performing associated display of the physiological sign parameters and the medication information on the monitor comprises:
associating the physiological sign parameters with the medication information in time, and then displaying on the monitor a correlation between a physiological sign parameter trend and a medication flow rate trend or a dose or concentration trend over time.

28. The method of claim 17, wherein performing associated display of the physiological sign parameters and the medication information on the monitor comprises:
associating physiological sign parameters within a preset time period before medication with the physiological sign parameters within a preset time period after the medication, and then displaying on the monitor an association between the physiological sign parameters and the use of the drug.

29. The method of claim 27 or 28, wherein the method further comprises:
presenting the physiological sign parameters by a max-min-average trend chart.

30. The method of claim 29, wherein forming of the max-min-average trend chart comprises:
obtaining maximum values, minimum values, and average values in a plurality of values of the physiological sign parameters measured in each unit time within the preset time period; and
connecting all maximum values, all minimum values, and all average values within the preset time period to form respective curves, so as to form the max-min-average trend chart representing a physiological sign parameter trend, wherein the preset time period comprises a number of unit times.

31. The method of claim 17, wherein the method further comprises the following step:
collecting time period-based statistics on infusion amounts of different drugs pumped into the body of the patient to obtain cumulative infusion amounts of different drugs in different time periods.

32. The method of claim 17, wherein the method further comprises the following step:
collecting statistics on types of abnormality events, the number of occurrences of the abnormality events, and key indicators in the abnormality events within a preset time period, and controlling to display the statistics.
